(19) Europäisches Patentamt / European Patent Office / Office européen des brevets

(11) **EP 2 638 869 B1**

(12) **FASCICULE DE BREVET EUROPEEN**

(45) Date de publication et mention
de la délivrance du brevet:
**24.07.2019 Bulletin 2019/30**

(51) Int Cl.:
***A61B 17/11*** *(2006.01)*

(21) Numéro de dépôt: **13159158.8**

(22) Date de dépôt: **14.03.2013**

(54) **Dispositif pour la mise en place d'une prothèse dans un conduit corporel**

Vorrichtung zum Einsetzen einer Prothese in einen Körperkanal

Device for placing a prosthesis in a body duct

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **15.03.2012 FR 1252339**

(43) Date de publication de la demande:
**18.09.2013 Bulletin 2013/38**

(73) Titulaire: **Abou Taam, Salam
75017 Paris (FR)**

(72) Inventeur: **Abou Taam, Salam
75017 Paris (FR)**

(74) Mandataire: **Ipside
7-9 Allées Haussmann
33300 Bordeaux Cedex (FR)**

(56) Documents cités:
**WO-A1-2004/058078      WO-A1-2011/020099
WO-A2-03/026475      US-A- 5 941 908
US-A- 6 036 702      US-A1- 2002 082 614
US-A1- 2005 267 498      US-A1- 2006 025 788
US-B1- 6 666 873**

**Description**

La présente invention concerne un dispositif pour la mise en place d'une prothèse dans un conduit corporel. Elle concerne également un ensemble d'implantation d'une prothèse dans un conduit corporel comprenant un tel dispositif et une unité de greffage contenant cette prothèse.

Il est connu de réaliser une suture manuelle chirurgicale pour réaliser une anastomose termino-terminale, c'est-à-dire réaliser une anastomose entre deux segments destinés à s'aboucher par leur extrémité libre à plein canal. Ce type d'anastomose qui est le plus fréquent en chirurgie vasculaire, permet de répondre à la majorité des situations cliniques de réparation artérielle.

Cependant, on observe que le temps opératoire nécessaire à la réalisation d'une telle suture manuelle est relativement long.

Or, lors d'une réparation vasculaire par exemple, l'anastomose nécessite un clampage vasculaire pour éviter toute hémorragie. La durée de ce clampage d'une part, constitue une épreuve d'effort cardiaque pour le patient pouvant conduire à un syndrome coronarien et d'autre part, génère une ischémie des tissus vascularisés par l'axe artériel d'aval.

En outre, une telle intervention manuelle impose une large accessibilité afin que le praticien puisse réaliser les gestes opératoires nécessaires à la liaison des deux segments entre eux. Cette accessibilité est aussi déterminante que la dextérité du praticien dans le bon accomplissement de la suture.

Toutefois, une telle accessibilité ne peut être obtenue qu'au travers d'une surface opératoire exposée importante, laquelle entraîne par exemple dans le cadre de la chirurgie aortique, des répercussions négatives sur les douleurs postopératoires, la reprise du transit intestinal, les pneumopathies, les coronaropathies et les durées de séjour hospitalier.

La chirurgie conventionnelle par mini-abord, ainsi que la chirurgie aortique laparoscopique, permettent de résoudre de tels inconvénients et de diminuer les répercussions sus-citées pour le patient.

Néanmoins, la chirurgie conventionnelle par mini-abord et la chirurgie aortique laparoscopique offrent des conditions d'accessibilité réduites, rendant difficile la réalisation des gestes chirurgicaux nécessaires à la suture vasculaire.

Il conviendrait donc de trouver une méthode d'anastomose autorisant la suture entre deux segments souples dans des conditions d'accessibilité réduite.

On connaît, par ailleurs, un dispositif de liaison mécanique permettant de relier un conduit corporel d'un patient et une prothèse tubulaire dont une extrémité est intubée dans ce conduit corporel.

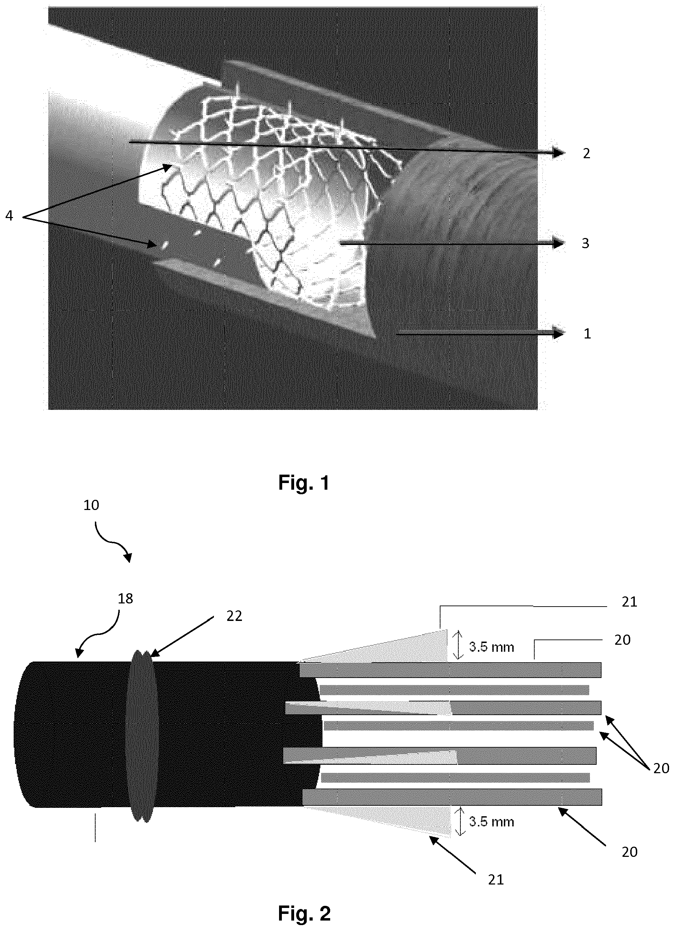

La Figure 1 montre un tel dispositif de liaison de l'état de l'art, encore connu sous le nom de "connecteur". Le conduit corporel 1 et la prothèse 2 sont ici montrés partiellement en coupe pour laisser apparaître le dispositif de liaison 3.

Ce dispositif de liaison 3 est ici un manchon expansible constitué d'un maillage et comportant au niveau de sa surface extérieure des picots 4 aptes à perforer la prothèse 2 et le conduit corporel 1 pour assurer la liaison entre ces deux éléments. Le manchon qui est en acier, a une longueur typiquement de l'ordre 20 mm.

L'architecture même de ce dispositif de liaison est conçue pour préserver la vascularisation de la paroi artérielle grâce à la disposition hélicoïdale des picots 4.

Ces picots 4 présentent ici un profil hémostatique conféré, de préférence, par une base ayant un profil cylindrique suivi d'un profil tétraédrique, un des sommets formant alors la pointe du picot. Ce profil hémostatique permet avantageusement de s'assurer de l'absence de saignement.

On pourra avantageusement se reporter à la demande de brevet WO 2004/032800 qui décrit plus en détails un tel dispositif de liaison de l'art antérieur.

Bien que ce dispositif de liaison donne de très bons résultats sur les travaux expérimentaux (Salam Abou Taam et al. J. Vase. Surg. 55(1) : 210-5 ; 2012), il n'est pas mis en oeuvre en chirurgie conventionnelle par mini-abord notamment en raison de la difficulté de son mise en place dans le conduit corporel.

Il existe donc un besoin pressant pour un ancillaire facilitant l'installation d'un tel dispositif de liaison en chirurgie, notamment en chirurgie mini-invasive.

Toutefois, un tel ancillaire devrait répondre à un certain nombre d'exigences ou surmonter un certain nombre d'inconvénients.

Tout d'abord, la longueur des picots du manchon prises relativement aux dimensions globales de ce dernier est importante. Ces picots doivent en effet pénétrer la paroi prothétique et artérielle en vue de l'assemblage de ces deux éléments.

Ainsi, l'étanchéité et la résistance de l'anastomose sont obtenues grâce au retour élastique de l'aorte sur le dispositif de liaison et la prothèse ainsi solidarisés, et aux picots qui empêchent les déplacements longitudinaux.

Le rôle joué par les picots du dispositif de liaison étant essentiel, l'ancillaire ne doit pas conduire à une déformation ou à une altération de ceux-ci.

De plus, le praticien doit réaliser des mesures comparatives avant de procéder au positionnement définitif du dispositif de liaison dans la prothèse et le conduit corporel. En effet, seule une portion de la prothèse est destinée à être liée définitivement au conduit corporel, le dispositif de liaison venant non seulement assembler cette portion de la prothèse au conduit corporel mais également être ancré directement dans le conduit corporel. Ces mesures comparatives peuvent induire un risque d'erreur d'origine humaine.

Ces mesures étant, par ailleurs, effectuées sur des prothèses possédant une certaine élasticité longitudinale, cette dernière peut biaiser les mesures.

On a également constaté que la pratique clinique rend difficile le maintien en rectitude, requis pour les calculs de longueur, des prothèses dans des espaces confinés notamment en laparoscopie.

Tous ces éléments peuvent conduire à un défaut de positionnement de la prothèse et/ou du dispositif de liaison dans le conduit corporel amenant à une certaine fragilisation de l'anastomose ainsi réalisée.

Or, il est impératif que l'anastomose réponde à des conditions de résistance à la traction et à une étanchéité suffisante pour assurer la sécurité du patient.

Enfin, la rigidité de l'ensemble formé par la prothèse et le dispositif de liaison, rend son introduction dans le conduit corporel du patient particulièrement laborieux pour le praticien.

US2006/025788 A1 divulgue un dispositif pour la mise en place d'un prothèse dans un conduit corporel selon le préambule de la revendication 1.

L'objectif de la présente invention est donc de proposer un ancillaire, simple dans sa conception et dans son mode opératoire, permettant l'assemblage entre une artère et un substitut vasculaire, dans des conditions d'accessibilité réduite ou lors de la chirurgie laparoscopique tout en surmontant les inconvénients exposés ci-dessus.

Un autre objectif de cet ancillaire est de permettre au praticien d'installer rapidement une unité de greffage dans un conduit corporel pour réaliser l'anastomose, et réduire ainsi la durée du clampage artériel.

Rappelons qu'on entend par « clampage », l'interruption du flux sanguin dans un vaisseau devant permettre sa réparation ou son remplacement sans entraîner de pertes sanguines.

A cet effet, l'invention concerne un dispositif pour la mise en place d'au moins une prothèse dans un conduit corporel, ledit dispositif comprenant une extrémité distale pour recevoir au moins ladite prothèse.

Selon l'invention, ce dispositif comporte les caractéristiques de la revendication 1, entre autres:

- un ensemble de conformation pour maintenir la partie de ladite prothèse destinée à être introduite dans ledit conduit corporel dans un état radialement comprimé de manière à faciliter l'introduction de ladite partie de prothèse dans ledit conduit corporel, ledit ensemble de conformation étant placé à ladite extrémité distale,
- la portion du dispositif recevant la partie de la prothèse destinée à être placée à l'extérieur du conduit corporel ayant un diamètre au moins égal au diamètre D dudit conduit corporel de sorte que l'opérateur ait un repère pour n'installer dans ledit conduit corporel que la seule partie de la prothèse radialement comprimée par ledit ensemble de conformation,
- un moyen d'actionnement pour passer ledit ensemble de conformation entre une position active dans laquelle cet ensemble maintient la seule partie de ladite prothèse destinée à être introduite dans ledit conduit corporel dans un état radialement comprimé et une position de repos où ladite partie de prothèse est dans sa configuration initiale, c'est-à-dire dans un état non radialement comprimé.

L'ancillaire sert de vecteur à l'ensemble dispositif de liaison/prothèse et n'est utilisé que le temps de l'introduction de cet ensemble dans le conduit corporel par une de ses extrémités. Il n'y a donc avantageusement pas de contact prolongé de cet ancillaire avec les tissus biologiques.

Cet ancillaire permet avantageusement de réduire significativement le temps opératoire.

Dans différents modes de réalisation particuliers de ce dispositif, chacun ayant ses avantages particuliers et susceptibles de nombreuses combinaisons techniques possibles:

- ledit dispositif étant destiné à mettre en place dans le conduit corporel une unité de greffage comprenant ladite prothèse, un dispositif de liaison à picots à élargissement radial pour assembler au moins ladite prothèse et ledit conduit corporel et une enveloppe de protection de diamètre extérieur $\Phi$ qui est placée entre les picots du dispositif de liaison et ladite prothèse, au moins le bout de l'extrémité distale dudit dispositif définit, dans la position active dudit ensemble de conformation, un orifice de diamètre d tel que

  $D > d \geq \Phi$.

On s'assure ainsi avantageusement que l'ancillaire ne déforme pas, ni n'altère les picots du dispositif de liaison en quelque manière que ce soit, garantissant ainsi la qualité de l'assemblage du conduit corporel et de la prothèse.

De préférence, le dispositif comporte un moyen de libération de ladite enveloppe afin d'assurer le retrait de cette enveloppe après mise en place de ladite au moins une prothèse.

L'enveloppe de protection comporte une extrémité proximale de diamètre inférieur à son extrémité distale, cette dernière étant adaptée au diamètre du dispositif de liaison à picots de manière à coulisser ainsi avantageusement vers l'extérieur pour découvrir le dispositif de liaison, permettre l'inflation d'un ballon gonflable dont l'expansion assurera la mise en contact dispositif de liaison-prothèse.

Selon l'invention, ledit dispositif a un axe principal, ledit moyen d'actionnement comporte au moins un élément déplaçable en translation le long dudit axe pour ajuster la longueur de ladite partie de ladite prothèse destinée à être introduite dans ledit conduit corporel, et ledit ensemble de conformation comporte des pattes disposées de manière circonférentielle autour dudit axe principal.

De préférence, ces pattes sont disposées circonférentiellement à égale distance l'une de l'autre de manière à appliquer une pression uniforme sur au moins ladite prothèse.

Selon l'invention, le moyen d'actionnement est un anneau rigide mobile en translation, chacune de ces pattes comporte sur sa surface extérieure une saillie sur laquelle l'anneau vient s'engager de manière à déplacer l'extrémité de chacune desdites pattes vers l'intérieur dudit dispositif.

De manière avantageuse, cette saillie présente une face inclinée dont le point le plus haut, placé du côté de l'extrémité distale du dispositif, présente une hauteur par rapport au point le plus bas de cette saillie égale à la distance de déplacement des extrémités des pattes vers l'intérieur dudit dispositif.

- ledit dispositif ayant un axe principal, ledit ensemble de conformation comprend une pointe rigide creuse et fixe placée à l'extrémité distale dudit dispositif, ladite pointe définissant une ouverture de passage pour ladite au moins une prothèse, ledit ensemble de conformation comportant de plus des pattes disposées de manière circonférentielle autour dudit axe principal, chacune desdites pattes étant mobile entre une position de libération de ladite au moins une prothèse dans laquelle lesdites pattes se déplacent en s'écartant l'une de l'autre vers l'extérieur pour libérer ladite prothèse et une position rétractée dans laquelle une partie au moins desdites pattes est reçue dans le corps dudit dispositif, l'extrémité desdites pattes venant prendre appui sur la surface extérieure de ladite au moins une prothèse en maintenant la partie de la prothèse destinée à être introduite dans ledit conduit corporel dans un état radialement comprimé.

De préférence, ladite pointe ayant la forme d'un tronc de cône creux, chacune desdites pattes présente un épaulement suivi d'une portion inclinée.

Ainsi, cet épaulement forme une saillie destinée à servir d'appui de la patte correspondante sur la pointe lorsque cette patte est dans sa position rétractée, une partie de celle-ci étant alors reçue dans le corps du dispositif tandis que la portion inclinée placée à l'extérieur de la pointe contribue au maintien d'une partie de la prothèse dans un état radialement comprimé, les épaulements des pattes plaqués contre le pourtour de l'ouverture de la pointe empêchant tout retrait vers l'intérieur du corps du dispositif du reste desdites pattes.

L'invention concerne également un ensemble d'implantation d'une prothèse dans un conduit corporel comprenant un dispositif pour la mise en place d'au moins une prothèse tel que décrit précédemment et une unité de greffage comportant

- ladite prothèse,
- un dispositif de liaison à picots à élargissement radial pour assembler au moins la prothèse et ledit conduit corporel,
- une enveloppe de protection placée entre les picots du dispositif de liaison et ladite prothèse,
- un ballon d'inflation pour élargir radialement le dispositif de liaison à picots en vue d'assembler la prothèse et le conduit corporel, et
- un cathéter pour supporter ladite unité et permettre le gonflage dudit ballon d'inflation.

Dans différents modes de réalisation particuliers de ce dispositif, chacun ayant ses avantages particuliers et susceptibles de nombreuses combinaisons techniques possibles:

- ledit ensemble formé par le dispositif de liaison à picots, l'enveloppe de protection et le ballon d'inflation est placé en saillie à l'extrémité distale dudit dispositif de manière à assurer l'assemblage dudit dispositif de liaison et dudit conduit corporel.
- ledit ballon d'inflation est un ballon non compliant ou semi-compliant. Rappelons que la « compliance » d'un ballon est définie comme la modification de diamètre du ballon en fonction de la pression appliquée.
  La compliance d'un ballon qui est donnée en pourcentage, est égale à

$$100\% \times \frac{\text{Diamètre du ballon à haute atmosphère} - \text{Diamètre du ballon à basse atmosphère}}{\text{Diamètre du ballon à basse atmosphère}}$$

- le ballon d'inflation a une taille maximale après gonflage supérieure d'au moins un quart de diamètre au diamètre maximal du dispositif de liaison,
- le nez du ballon d'inflation est court de manière à ne pas augmenter la distance entre l'extrémité libre du conduit corporel et la zone de clampage du conduit corporel.

La présente invention concerne encore un ensemble d'implantation d'une prothèse dans un conduit corporel comprenant un dispositif pour la mise en place d'au moins une prothèse tel que décrit précédemment et une unité de greffage comportant ladite prothèse, un dispositif de liaison à picots à élargissement radial pour assembler au moins la prothèse et ledit conduit corporel, ledit dispositif de liaison étant auto expansible, une enveloppe de protection placée entre les picots du dispositif de liaison et ladite prothèse et un cathéter pour supporter ladite unité de greffage.

Les dispositifs de liaison à picots auto expansibles sont capables de s'expandre lors du retrait de l'enveloppe de protection dans laquelle ils sont contraints. A titre purement illustratif, ces dispositifs de liaison auto expansibles sont réalisés dans des matériaux à mémoire de forme tels que le Nitinol.

L'invention sera décrite plus en détail en référence aux dessins annexés dans lesquels:

- la figure 1 montre une vue partielle et élargie d'un conduit corporel dans lequel une prothèse a été introduite, cette prothèse étant reliée au conduit corporel par un dispositif de liaison à picots de l'état de l'art ;
- la figure 2 représente schématiquement un dispositif pour la mise en place d'au moins une prothèse dans un conduit corporel selon un mode de réalisation particulier de l'invention, ce dispositif étant représenté dans sa position de repos;
- la figure 3 montre le dispositif de la Figure 2 dans sa position activée;
- la figure 4 montre le dispositif de la Figure 2 dans lequel a été placé une unité de greffage;

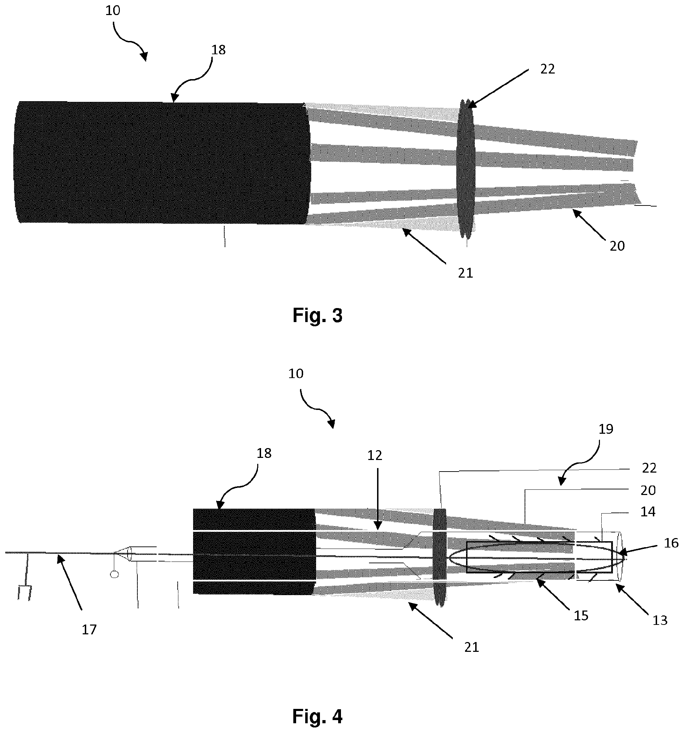

Les Figures 3 à 4 montrent de manière schématique un ancillaire 10 pour la mise en place d'une unité de greffage dans un conduit corporel selon un mode de réalisation préféré de la présente invention. Cet ancillaire est représenté dans sa position de repos (Figure 2) et dans sa position activée (Figure 3).

L'unité de greffage comporte en allant de l'extérieur vers le centre de cette unité :

- une prothèse, ou substitut vasculaire, 12,
- une enveloppe de protection 13,
- un dispositif de liaison 14 à picots à élargissement radial, ce dispositif comportant au niveau de sa surface extérieure des picots 15 aptes à perforer la prothèse 12 et le conduit corporel pour assurer la liaison de ces deux éléments,
- un ballon d'inflation 16 pour élargir radialement le dispositif de liaison à picots 14 en vue d'assembler la prothèse 12 et le conduit corporel,
- un cathéter 17 pour supporter l'unité de greffage et permettre l'inflation du ballon.

L'enveloppe de protection 13 qui est réalisée en polychlorure de vinyle (PVC) ou en polymère, est placée entre les picots 15 du dispositif de liaison et la prothèse 12 de manière à éviter un mouvement d'entrainement intempestif de cette dernière par l'accrochage des picots 15. Cette enveloppe de protection 13 forme ainsi une "chaussette" venant intégralement recouvrir le dispositif de liaison 14 à picots. La partie de l'enveloppe de protection 13 destinée à recouvrir le dispositif de liaison à picots 14 a un diamètre supérieur typiquement de 1 à 2 mm au diamètre du dispositif de liaison 14 à picots dans sa position radialement comprimée tandis que le reste de l'enveloppe de protection 13 présente un diamètre supérieur d'environ 1 mm au cathéter porteur 17 (Figure 4).

Cette enveloppe de protection 13 est rétractable pour permettre l'expansion radiale du dispositif de liaison 14 à picots.

L'ancillaire 10 permet la réalisation successive des étapes conduisant à l'anastomose entre un conduit corporel tel qu'une artère, et le substitut vasculaire 12, dans des conditions d'accessibilité réduite ou lors de la chirurgie laparoscopique. Ces étapes comprennent notamment le positionnement de la prothèse 12, du dispositif de liaison 14 à picots et le gonflement du ballon d'inflation 16 afin d'assurer l'assemblage de la prothèse 12 et du conduit corporel.

L'ancillaire 10 comprend une extrémité proximale 18 et une extrémité distale 19, cette dernière étant destinée à recevoir l'unité de greffage contenant la prothèse 12 à installer dans le conduit corporel.

L'extrémité proximale 18 de cet ancillaire est formée par une portion cylindrique creuse au bout de laquelle passe un certain nombre d'organes de commande permettant au praticien d'agir à distance sur l'unité de greffage durant l'opération. Un tel organe de commande est, par exemple, le cathéter 17 permettant l'inflation du ballon d'inflation 16 de l'unité de greffage.

Le diamètre extérieur de cette portion cylindrique creuse 18 est au moins égal au diamètre D non comprimé du conduit corporel dans lequel la prothèse 12 doit être installée.

L'ancillaire 10 comporte également un ensemble de conformation pour maintenir la partie de la prothèse 12 destinée à être introduite dans le conduit corporel dans un état radialement comprimé avant installation de manière à faciliter l'introduction de la partie correspondante de l'unité de greffage dans ce conduit corporel. La compression du dispositif de liaison à picots est réalisée sur l'enveloppe de protection 13 afin d'éviter un contact indésirable avant l'expansion du dispositif de liaison 14.

Cet ensemble de conformation autorise en outre avantageusement une réversibilité de la compression du substitut vasculaire, ou prothèse, 12, lequel peut recouvrer sa forme initiale à l'arrêt de la compression grâce à ses propriétés de déformation élastique. A titre illustratif, cette déformabilité existe à la fois pour les prothèses en polytétrafluoroéthylène (PTFE) ou en polytéréphtalate d'éthylène (PET). Ces propriétés sont à la base de leur utilisation en chirurgie vasculaire, afin de reproduire les propriétés élastiques d'une artère native et diminuer ainsi les risques thrombotiques.

L'ensemble de conformation comporte huit (8) lamelles 20 longitudinales disposées circonférentiellement en formant ainsi un prolongement de la portion cylindrique creuse 18. Ces lamelles qui ont ici une longueur de 30 mm environ, sont réalisées en polychlorure de vinyle (PVC). Ces lamelles 20 sont placées à égale distance les unes des autres de manière à appliquer une pression uniforme sur la prothèse de l'unité de greffage en vue d'obtenir une déformation homogène de cette prothèse.

Chacune de ces lamelles 20 est pourvue sur sa surface extérieure d'une protubérance 21 présentant une face inclinée, ou pente, dont le point le plus haut, est placé du côté du bout de l'extrémité distale de l'ancillaire et le point le plus bas est placé du côté de la portion cylindrique creuse 18. Le point le plus haut de cette saillie 21 présente une hauteur par rapport à son point le plus bas égale à la distance de déplacement des extrémités des pattes vers l'intérieur de l'ancillaire.

Par ailleurs, ces protubérances 21 sont placées sur les lamelles 20 de sorte que seule la partie de la prothèse 12 destinée à être placée à l'intérieur du conduit corporel est radialement comprimée pour faciliter son installation dans le conduit corporel. Le reste de la prothèse qui est destinée à être placé à l'extérieur du conduit corporel conserve un diamètre égal au diamètre D du conduit corporel de sorte que l'opérateur ait un repère pour n'installer dans ledit conduit corporel que la seule partie de la prothèse radialement comprimée.

L'ancillaire comporte un anneau rigide 22 mobile en translation le long de la portion cylindrique creuse 18 et des lamelles 20. Le déplacement de cet anneau rigide 22 en direction de l'extrémité distale 19 de l'ancillaire permet d'activer la déformation de la seule partie de la prothèse 12 destinée à être placée à l'intérieur du conduit corporel. L'anneau 22 ainsi déplacé manuellement par le praticien, vient s'engager sur les faces inclinées des protubérances 21 et progressivement imposer une diminution de l'orifice délimité par l'extrémité des lamelles 20.

Avantageusement, seule la prothèse 12 de cette unité est radialement comprimée, l'enveloppe protectrice 13 recouvrant le dispositif de liaison 14 à picots n'étant pas affectée de manière à éviter toute déformation ou altération de ces picots.

L'extrémité distale de la prothèse 12 peut ainsi subir une inflexion centripète assurant une diminution du diamètre d'au moins 30% sur ses 15 derniers mm, laquelle correspond à la longueur de la prothèse devant être introduite dans le conduit corporel. Une réduction du diamètre de 30% nécessite une division du diamètre d'un facteur 0,7. Pour obtenir cette diminution du diamètre interne, les lamelles 20 sont triangulaires sur 15 mm avant d'être planes sur les 15 derniers mm. A titre d'exemple, pour un ancillaire à destination aortique, la hauteur du triangle est de 3,5 mm étudiée pour une réduction minimale du diamètre interne à 15 mm.

Comme le montre la Figure 4, l'ensemble constitué de l'enveloppe de protection 13, du dispositif de liaison 14 à picots et du ballon d'inflation 16 dépasse de la prothèse d'une distance correspondant à la distance sur laquelle le dispositif de liaison 14 doit être en contact direct avec le conduit corporel. Ici, cette distance d'ancrage du dispositif de liaison est de 5 mm.

Cette réduction du diamètre permet à l'ancillaire de faire pénétrer la prothèse 12 dans l'artère de même diamètre. Le retrait de la gaine s'effectue après retrait de l'anneau 22 coulissant. Ceci permet aux lamelles 20 de récupérer leur disposition initiale afin d'éviter le retrait involontaire de la prothèse 12. Une fois les lamelles 20 ouvertes, l'enveloppe protectrice 13 du dispositif est retirée jusqu'à la garde. Le ballon d'inflation 16 peut alors être gonflé jusqu'à sa pression nominale (pour le diamètre souhaité) appliquant ainsi le dispositif de liaison 14 à picots à la prothèse 12 et à la paroi du conduit corporel.

Le ballon d'inflation 16 utilisé a un diamètre égal après gonflage au diamètre du conduit corporel de manière à assurer le plaquage du dispositif de liaison 14 à picots contre la paroi de ce conduit corporel. La longueur de ce ballon d'inflation 16 correspond à celle des lamelles 20.

De préférence, le ballon d'inflation 16 est un ballon semi-compliant. Par exemple, le ballon Opta® Pro commercialisé par la société Cordis, Pays-Bas, et qui est un ballon semi-compliant en polyuréthane, est adapté à la présente invention. La force radiale des ballons doit être suffisante pour permettre au dispositif de liaison 14 à picots d'atteindre son diamètre nominal.

Le nez du ballon d'inflation 16 est, de préférence, le plus court possible pour ne pas augmenter le collet nécessaire au clampage, c'est-à-dire la distance entre l'extrémité du segment artériel et la zone de clampage.

De préférence, le cathéter 17 est pourvu sur sa surface extérieure de repères visuels ou d'une graduation telle qu'une graduation millimétrique permettant au praticien de pré-positionner les différents éléments de l'unité de greffage garantissant ainsi un placement optimal de ces éléments dans le conduit corporel et ce, sans effort.

Avantageusement, le principe de ce type d'ancillaire repose sur la nécessité d'obtenir une diminution réversible du diamètre de l'extrémité de l'ancillaire d'au moins 30 %. Ainsi, le diamètre externe de l'ancillaire pourrait passer de 22 à 15,4 mm à l'étage aortique, de 12 à 8,4 mm à l'étage iliaque et de 8 à 5,6 mm à l'étage fémoral. Avec ces caractéristiques, 3 gammes de diamètre d'ancillaires permettraient de répondre à la majorité des situations cliniques.

## Revendications

1. Dispositif pour la mise en place d'au moins une prothèse (12) dans un conduit corporel, ledit dispositif comprenant une extrémité distale pour recevoir au moins ladite prothèse (12), ledit dispositif comportant

   - un ensemble de conformation (20, 21) pour maintenir la partie de ladite prothèse (12) destinée à être introduite dans ledit conduit corporel dans un état radialement comprimé de manière à faciliter l'introduction de ladite partie de prothèse (12) dans ledit conduit corporel, ledit ensemble de conformation (20, 21) étant placé à ladite extrémité distale,
   - la portion du dispositif recevant la partie de la prothèse (12) destinée à être placée à l'extérieur du conduit corporel ayant un diamètre au moins égal au diamètre D dudit conduit corporel de sorte que l'opérateur ait un repère pour n'installer dans ledit conduit corporel que la seule partie de la prothèse (12) radialement comprimée,
   - un moyen d'actionnement (22) pour passer ledit ensemble de conformation (20, 21) entre une position active dans laquelle cet ensemble maintient la seule partie de ladite prothèse (12) destinée à être introduite dans ledit conduit corporel dans un état radialement comprimé et une position de repos où ladite partie de prothèse (12) est dans sa configuration initiale, ledit dispositif ayant un axe principal, ledit ensemble de conformation (20, 21) comportant des pattes (20) disposées de manière circonférentielle autour dudit axe principal, ledit moyen d'actionnement (22) étant un anneau rigide mobile en translation, **caractérisé en ce que** chacune desdites pattes (20) comporte sur sa surface extérieure une saillie (21) sur laquelle l'anneau (22) vient s'engager de manière à déplacer l'extrémité de chacune desdites pattes vers l'intérieur dudit dispositif.

2. Dispositif selon la revendication 1, **caractérisé en ce que** ledit moyen d'actionnement (22) comporte au moins un élément déplaçable en translation le long dudit axe pour ajuster la longueur de ladite partie de ladite prothèse (12) destinée à être introduite dans ledit conduit corporel.

3. Dispositif selon la revendication 1, **caractérisé en ce que** lesdites pattes (20) sont disposées circonférentiellement à égale distance l'une de l'autre de manière à appliquer une pression uniforme sur ladite prothèse (12).

4. Dispositif selon la revendication 1, **caractérisé en ce que** ladite saillie (21) présente une face inclinée dont le point le plus haut, placé du côté de l'extrémité distale du dispositif, présente une hauteur par rapport au point le plus bas de cette saillie égale à la distance de déplacement des extrémités des pattes vers l'intérieur dudit dispositif.

5. Ensemble d'implantation d'une prothèse (12) dans un conduit corporel comprenant un dispositif selon l'une quelconque des revendications 1 à 4 et une unité de greffage comportant ladite prothèse (12), un dispositif de liaison (14) à picots à élargissement radial pour assembler au moins la prothèse (12) et ledit conduit corporel, une enveloppe de protection (13) placée entre les picots (15) du dispositif de liaison et ladite prothèse (12), un ballon d'inflation (16) pour élargir radialement le dispositif de liaison (14) à picots en vue d'assembler la prothèse (12) et le conduit corporel et un cathéter pour supporter ladite unité et permettre le gonflage dudit ballon d'inflation.

6. Ensemble selon la revendication 5, **caractérisé en ce que** l'ensemble formé par le dispositif de liaison (14) à picots, l'enveloppe de protection (13) et le ballon d'inflation (16) est placé en saillie à l'extrémité distale dudit dispositif de manière à assurer l'assemblage dudit dispositif de liaison et dudit conduit corporel.

7. Ensemble selon la revendication 5 ou 6, **caractérisé en ce que** ledit ballon d'inflation (16) est un ballon semi-compliant ou non compliant.

8. Ensemble selon l'une quelconque des revendications 5 à 7, **caractérisé en ce que** le nez du ballon d'inflation (16) est court de manière à ne pas augmenter la distance entre l'extrémité libre du conduit corporel et la zone de clampage du conduit corporel.

9. Ensemble d'implantation d'une prothèse (12) dans un conduit corporel comprenant un dispositif selon l'une quelconque des revendications 1 à 4 et une unité de greffage comportant ladite prothèse (12), un dispositif de liaison (14) à picots à élargissement radial pour assembler au moins la prothèse (12) et ledit conduit corporel, ledit dispositif de liaison étant auto expansible, une enveloppe de protection (13) placée entre les picots (15) du dispositif de liaison et ladite prothèse (12) et un cathéter pour supporter ladite unité.

**Patentansprüche**

1. Vorrichtung zum Einsetzen von mindestens einer Prothese (12) in eine Körperröhre, wobei die Vorrichtung ein distales Ende umfasst, um mindestens die Prothese (12) aufzunehmen, wobei die Vorrichtung umfasst

   - eine Formgebungsanordnung (20, 21), um den Teil der Prothese (12), der dazu bestimmt ist, in die Körperröhre eingeführt zu werden, in einem radial komprimierten Zustand zu halten, um das Einführen des Prothesenteils (12) in die Körperröhre zu erleichtern, wobei die Formgebungsanordnung (20, 21) am distalen Ende platziert ist, wobei der Abschnitt der Vorrichtung, der den Teil der Prothese (12) aufnimmt, der dazu bestimmt ist, außerhalb der Körperröhre platziert zu werden, einen Durchmesser aufweist, der mindestens gleich dem Durchmesser D der Körperröhre ist, sodass der Bediener einen Anhaltspunkt besitzt, um nur den Teil der Prothese (12), der radial komprimiert ist, in der Körperröhre zu installieren,
   - ein Betätigungsmittel (22), um die Formgebungsanordnung (20, 21) zwischen einer aktiven Stellung, in der diese Anordnung nur den Teil der Prothese (12), der dazu bestimmt ist, in die Körperröhre eingeführt zu werden, in einem radial komprimierten Zustand hält, und einer Ruhestellung zu überführen, in der sich der Prothesenteil (12) in seiner Ausgangskonfiguration befindet, wobei die Vorrichtung eine Hauptachse aufweist, wobei die Formgebungsanordnung (20, 21) Ansätze (20) umfasst, die umfangsmäßig um die Hauptachse herum angeordnet sind, wobei das Betätigungsmittel (22) ein translationsbeweglicher starrer Ring ist, **dadurch gekennzeichnet, dass** jeder der Ansätze (20) an seiner Außenfläche einen Vorsprung (21) umfasst, an dem der Ring (22) eingreift, um das Ende jedes der Ansätze zur Innenseite der Vorrichtung zu verschieben.

2. Vorrichtung nach Anspruch 1, **dadurch gekennzeichnet, dass** das Betätigungsmittel (22) mindestens ein Element umfasst, das entlang der Achse translationsverschieblich ist, um die Länge des Teils der Prothese (12), der dazu bestimmt ist, in die Körperröhre eingeführt zu werden, anzupassen.

3. Vorrichtung nach Anspruch 1, **dadurch gekennzeichnet, dass** die Ansätze (20) in Umfangsrichtung in gleicher Distanz voneinander angeordnet sind, um einen gleichmäßigen Druck auf die Prothese (12) auszuüben.

4. Vorrichtung nach Anspruch 1, **dadurch gekennzeichnet, dass** der Vorsprung (21) eine schräge Fläche aufweist, deren höchster Punkt, der auf der Seite des distalen Endes der Vorrichtung platziert ist, eine Höhe im Verhältnis zum niedrigsten Punkt dieses Vorsprungs aufweist, die gleich der Verschiebungsungsdistanz der Enden der Ansätze zur Innenseite der Vorrichtung ist.

5. Anordnung zum Implantieren einer Prothese (12) in eine Körperröhre, die eine Vorrichtung nach einem der Ansprüche 1 bis 4 und eine Transplantationseinheit umfasst, welche die Prothese (12), eine Verbindungsvorrichtung (14) mit Widerhaken mit radialer Aufweitung, um mindestens die Prothese (12) und die Körperröhre zusammenzufügen, eine Schutzhülle (13), die zwischen den Widerhaken (15) der Verbindungsvorrichtung und der Prothese (12) platziert ist, einen Inflationsballon (16), um die Verbindungsvorrichtung (14) mit Widerhaken im Hinblick darauf, die Prothese (12) und die Körperröhre zusammenzufügen, radial aufzuweiten, und einen Katheter umfasst, um die Einheit zu stützen und das Aufblasen des Inflationsballons zu ermöglichen.

6. Anordnung nach Anspruch 5, **dadurch gekennzeichnet, dass** die von der Verbindungsvorrichtung (14) mit Widerhaken, der Schutzhülle (13) und dem Inflationsballon (16) gebildete Anordnung am distalen Ende der Vorrichtung vorspringen platziert wird, um das Zusammenfügen der Verbindungsvorrichtung und der Körperröhre sicherzustellen.

7. Anordnung nach Anspruch 5 oder 6, **dadurch gekennzeichnet, dass** der Inflationsballon (16) ein halbnachgiebiger oder nicht nachgiebiger Ballon ist.

8. Anordnung nach einem der Ansprüche 5 bis 7, **dadurch gekennzeichnet, dass** die Spitze des Inflationsballons (16) kurz ist, um die Distanz zwischen dem freien Ende der Körperröhre und dem Verklammerungsbereich der Körperröhre nicht zu erhöhen.

9. Anordnung zum Implantieren einer Prothese (12) in eine Körperröhre, die eine Vorrichtung nach einem der Ansprüche 1 bis 4 und eine Transplantationseinheit umfasst, welche die Prothese (12), eine Verbindungsvorrichtung (14) mit Widerhaken mit radialer Aufweitung, um mindestens die Prothese (12) und die Körperröhre zusammenzufügen, wobei die Verbindungsvorrichtung selbstexpandierend ist, eine Schutzhülle (13), die zwischen den Widerhaken (15) der Verbindungsvorrichtung und der Prothese (12) platziert ist, und einen Katheter umfasst, um die Einheit zu stützen.

**Claims**

1. Device for placing at least one prosthesis (12) in a corporeal duct, said device comprising a distal end for receiving at least said prosthesis (12), said device comprising

   - a conforming assembly (20, 21) for maintaining the part of said prosthesis (12) that is intended to be inserted into said corporeal duct in a radially compressed state so as to facilitate the insertion of said prosthesis part (12) inside said corporeal duct, said conforming assembly (20, 21) being placed at said distal end, the portion of the device receiving the part of the prosthesis (12) that is intended to be placed outside of the corporeal duct having a diameter that is at least equal to the diameter D of said corporeal duct such that the operator has a marker ensuring that only the sole part of the prosthesis (12) that is radially compressed is inserted into said corporeal duct,
   - an actuating means (22) for passing said conforming assembly (20, 21) between an active position wherein said assembly maintains the sole part of said prosthesis (12) that is intended to be inserted into said corporeal duct in a radially compressed state and a rest position wherein said prosthesis part (12) is in the initial configuration thereof, said device having a major axis, said conforming assembly (20, 21) comprising tabs (20) that are disposed circumferentially about said major axis, said actuating means (22) being a rigid ring capable of moving in translation, **characterised in that** each of said tabs (20) comprises, on the outer surface thereof, a protrusion (21) onto which the ring (22) is engaged in order to move the end of each of said tabs towards the interior of said device.

2. Device according to claim 1, **characterised in that** said actuating means (22) comprises at least one element that is capable of being moved in translation along said axis in order to adjust the length of said part of said prosthesis (12) that is intended to be inserted into said corporeal duct.

3. Device according to claim 1, **characterised in that** said tabs (20) are disposed circumferentially at an equal distance from one another in order to apply a uniform pressure to said prosthesis (12).

4. Device according to claim 1, **characterised in that** said protrusion (21) has an inclined face, the highest point whereof, placed on the side of the distal end of the device, has a height relative to the lowest point of said protrusion that is equal to the moving distance of the ends of the tabs towards the interior of the device.

5. Assembly for implanting a prosthesis (12) in a corporeal duct comprising a device according to any of claims 1 to 4, and a grafting unit comprising said prosthesis (12), a spurred connecting device (14) with radial enlargement for assembling at least the prosthesis (12) with said corporeal duct, a protective enclosure (13) placed between the spurs (15) of the connecting device and said prosthesis (12), an inflation balloon (16) for radially enlarging the spurred connecting device (14) in order to assemble the prosthesis (12) with the corporeal duct, and a catheter for supporting said unit and for allowing said inflation balloon to be inflated.

6. Assembly according to claim 5, **characterised in that** the assembly formed by the spurred connecting device (14), the protective enclosure (13) and the inflation balloon (16) is placed such that it projects at the distal end of said device so as to ensure the assembly of said connecting device with said corporeal duct.

7. Assembly according to claim 5 or 6, **characterised in that** said inflation balloon (16) is a semi-compliant or non-compliant balloon.

8. Assembly according to any of claims 5 to 7, **characterised in that** the port of the inflation balloon (16) is short so as not to increase the distance between the free end of the corporeal duct and the clamping area of the corporeal duct.

9. Assembly for implanting a prosthesis (12) in a corporeal duct comprising a device according to any of claims 1 to 4, and a grafting unit comprising said prosthesis (12), a spurred connecting device (14) with radial enlargement for assembling at least the prosthesis (12) with said corporeal duct, said connecting device being self-expandable, a protective enclosure (13) placed between the spurs (15) of the connecting device and said prosthesis (12) and a catheter for supporting said unit.

**Fig. 1**

**Fig. 2**

**Fig. 3**

**Fig. 4**

## RÉFÉRENCES CITÉES DANS LA DESCRIPTION

**Documents brevets cités dans la description**

- WO 2004032800 A

- US 2006025788 A1

**Littérature non-brevet citée dans la description**

- **SALAM ABOU TAAM et al.** *J. Vase. Surg.,* 2012, vol. 55 (1), 210-5